# EUROPÄISCHE PATENTSCHRIFT

(11) **EP 2 294 046 B1**
(45) Veröffentlichungstag und Bekanntmachung des Hinweises auf die Patenterteilung: **01.02.2012**
(21) Anmeldenummer: 09753744.3
(22) Anmeldetag: 16.04.2009
(51) Int. Cl.: C07C 51/36, C07C 51/353, C07C 51/43, C07C 51/44, C07C 55/21, C07C 57/13

(54) **VERFAHREN ZUR HERSTELLUNG VON DODECA-2,10-DIEN-1,12-DICARBONSÄURE BZW. 1,12-DODECANDICARBONSÄURE MITTELS RING-ÖFFNENDER KREUZMETATHESE (ROCM) VON CYCLOOCTEN MIT ACRYLSÄURE**
METHOD FOR PRODUCING DODECA-2,10-DIENE-1,12-DICARBOXYLIC ACID OR 1,12-DODECANE DICARBOXYLIC ACID BY WAY OF RING-OPENING CROSS-METATHESIS (ROCM) OF CYCLOOCTENE WITH ACRYLIC ACID
PROCÉDÉ DE PRODUCTION D'ACIDE DODÉCA-2,10-DIÈNE-1,12-DICARBOXYLIQUE OU D'ACIDE 1,12-DODÉCANE DICARBOXYLIQUE PAR OUVERTURE DE CYCLE PAR MÉTATHÈSE CROISÉE (ROCM) DU CYCLOOCTÈNE AVEC L'ACIDE ACRYLIQUE

(30) Priorität: 30.05.2008 DE 102008002092
(43) Veröffentlichungstag der Anmeldung: 16.03.2011
(73) Patentinhaber: Evonik Degussa GmbH, 45128 Essen (DE)
(72) Erfinder: HANNEN, Peter, 45659 Recklinghausen (DE); ROOS, Martin, 45721 Haltern am See (DE); HÄGER, Harald, 59348 Lüdinghausen (DE)
(86) Internationale Anmeldenummer: PCT/EP2009/054519
(87) Internationale Veröffentlichungsnummer: WO 2009/144089

(56) Entgegenhaltungen:
- I. SAITO; R. NEGATA; K. YUBA; T. MATSUURA: "Synthesis of Synthetic alpha,omega-Dicarboxylic Acids and Unsaturated Carboxylic Acids from Silyl Enol Ethers" TETRAHEDRON LETTERS, Bd. 24, Nr. 41, 1983, Seiten 4439-4442, XP002529873
- RANDL S ET AL: "Ring opening-cross metathesis of unstrained cycloalkenes" CHEMICAL COMMUNICATIONS - CHEMCOM, ROYAL SOCIETY OF CHEMISTRY, GB, 4. November 2001 (2001-11-04), Seiten 1796-1797, XP002334391 ISSN: 1359-7345 in der Anmeldung erwähnt

## Beschreibung

Alkyldicarbonsäuren stellen wichtige Verbindungen für die Herstellung von Kunststoffen wie Polyestern und Polyamiden dar. Besonders zu erwähnen sind dabei Hochleistungskunststoffe auf Basis von Polyamiden, wie sie z.B. für die Herstellung von Treibstoffleitungen verwendet werden. Eine dieser Dicarbonsäuren ist die 1,12-Dodecandicarbonsäure (DDS). Als großtechnische Verfahren zur Herstellung von DDS sind vor allem zu nennen:
- Spaltung von Cyclododecanol mit konzentrierter Salpetersäure; Invista und Degussa
- Oxidative Kupplung und Öffnung von Cyclohexanon mit H₂O₂; Ube
- Biotechnologische Verfahren, in dem n-Dodekan endständig zu DDS oxidiert wird; Cathay Biotechnology und andere

Diese Verfahren sind energieintensiv und produzieren große Mengen Abfall, der aufwendig entsorgt werden muss. Zudem ist die Selektivität oftmals nicht groß. So ist z.B. bei der oxidativen Spaltung von Cyclododecanol mit Salpetersäure die Bildung kürzerkettiger 'Bruchsäuren' durch oxidativen Abbau ein Problem.

SAITO; R. NEGATA; K. YUBA; T. MATSUURA: "Synthesis of Synthetic alpha,omega-Dicarboxylic Acids and Unsaturated Carboxylic Acids from Silyl Enol Ethers" TETRAHEDRON LETTERS, Bd. 24, Nr. 41, 1983, Seiten 4439-4442, XP002529873 offenbart ein Verfahren zur Herstellung von Dodeca-2,10-dien-1,12-dicarbonsäure ausgehend von Cyclohexenon über einen Silylenolether.

Einen neuen Ansatz zur Herstellung von Dicarbonsäuren stellt die Metathese dar. Über eine Kombination von Ring-öffnender-Metathese (ROM) mit Kreuz-metathese (CM) lassen sich unter Verwendung geeigneter Prä-Katalysatoren aus Cyclalkenen und Acrylsäure in einem Schritt aliphatische α,β-ungesättigte Dicarbonsäuren herstellen. Setzt man als Edukt Cycloocten (1) mit Acrylsäure (2) um, so wird Dodecan-2,10-dien-1,12-dicarbonsäure (3) erhalten (Schema 1). Die Kombination beider Reaktionsschritte wird auch als ROCM-(ring opening/cross-metathesis) oder ROX-Metathese bezeichnet.

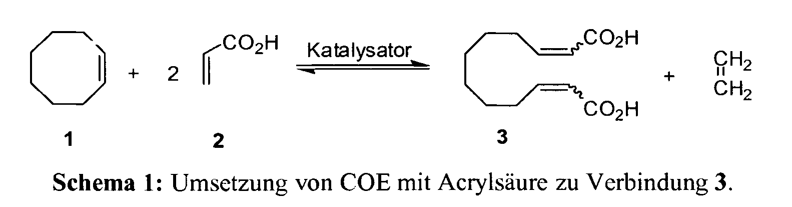

Die erhaltene α,β-ungesättigte Dicarbonsäure lässt sich in einem zweiten Schritt hydrieren, wobei die gewünschte 1,12-Dodecandicarbonsäure (4) erhalten wird (Schema 2).

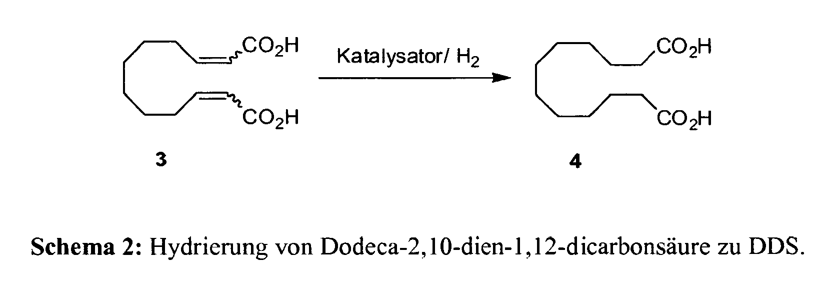

Es ist allerdings auch denkbar, die ungesättigte Dicarbonsäure als Monomer zur Herstellung ungesättigter Polyamide oder Polyester einzusetzen. Die so erhaltenen Polymere ließen sich im Nachhinein vernetzen was für viele Anwendungen interessant ist.

Bei Metathese-Reaktionen handelt es sich um Gleichgewichtsreaktionen mit einer entsprechenden Produktverteilung. Die oben beschriebene Reaktion kann verschiedene Reaktionswege einschlagen. So kann das Cycloalken im Sinne einer Ring-öffnenden-Metathese-Polymerisation zu einem Polyolefin reagieren. Des Weiteren kann es zur Bildung verschiedener telechelen Oligomere kommen. Diese Nebenreaktionen wirken sich negativ auf die Ausbeute an gewünschtem Produkt aus und erschweren zudem eine Aufarbeitung des Reaktionsgemisches. Durch die Bildung von Ethylen, das als Gas der flüssigen Phase entzogen werden kann, kommt es zur Verschiebung des Gleichgewichtes zugunsten des Produktes. Dieser Effekt reicht allerdings nicht aus, um Nebenreaktionen wie die Polymerisation vollständig zu unterbinden.

### Stand der Technik

### Beim bisherigen Stand der Technik

**(**Morgan, John, P., Morrill, Christie; Grubbs, Robert, H.; Choi, Tae-Lim WO 02/079127 A1**.**
Choi, T-L.; Lee, C. W.; Chatterjee, A. K.; Grubbs, R. H. J. Am. Chem. Soc. 2001,123, 10417-10418**.**
Randl, S.; Connon, S. J.; Blechert, S. J. Chem. Soc., Chem. Commun. 2001, 1796-1797**)**
wird in verdünnten Lösungen (c ~ 0,2 M) gearbeitet um die Reaktion in Richtung der niedermolekularen Produkte zu drängen. Zur Aufarbeitung des Reaktionsgemisches und Abtrennung des Katalysators kommt überwiegend die Säulenchromatographie mit Kieselgel zum Einsatz. Des Weiteren wird als Lösungsmittel überwiegend Dichlormethan eingesetzt, das im Hinblick auf eine großtechnische Umsetzung als problematisch anzusehen ist. Das Arbeiten in großer Verdünnung, der Verbrauch großer Mengen Lösungsmittel bei der Aufarbeitung und der Einsatz gesundheitsschädlicher Lösungsmittel stehen dem Konzept eines nachhaltigen Verfahrens, das einer technischen Umsetzung zugrunde liegen sollte, entgegen.

Es wurde nun überraschenderweise gefunden, dass es durch ein Verfahren gemäß den Ansprüchen möglich ist, das Gleichgewicht vollständig in Richtung des gewünschten Produktes zu verschieben ohne in großer Verdünnung zu arbeiten. Zudem wird durch das beschriebene Verfahren ein effektives Recyceln des Katalysators möglich. Dies wird erreicht indem man, im Gegensatz zur bisherigen Praxis, bei hohen Substratkonzentrationen von 1 Mol/L Cycloocten bis hin zu Reaktionen in Substanz arbeitet. Im Laufe der Reaktion fällt bei Überschreiten des Löslichkeitsproduktes die α,β-ungesättigte Dicarbonsäure aus und wird somit dem Gleichgewicht (in der homogenen Phase) entzogen. Neben der Gleichgewichtsverschiebung in Richtung des gewünschten Produktes durch Bildung von gasförmigen Ethylen kommt hier ein zweiter positiver Effekt hinzu.

Eine weitere Herausforderung stellt die Abtrennung und ggf. das Recyceln des Katalysators dar. Oft muss der Katalysator durch Säulenchromatographie aufwendig abgetrennt werden, was eine technische Umsetzung unrentabel macht.

Dadurch, dass bei dem hier beschriebenen Verfahren das Produkt als Feststoff anfällt, kann dieses leicht abfiltriert, gereinigt und der im Filtrat gelöste Katalysator recycelt werden.

Die beschriebene Reaktion wird bei Temperaturen von 10 bis 100 °C, bevorzugt bei 20 bis 80 °C und besonders bevorzugt bei 20 bis 60 °C durchgeführt.

Die beschriebene Reaktion lässt sich in Substanz als auch unter Verwendung eines Lösungsmittels durchführen. Als Lösungsmittel eignen sich acyclische als auch cyclische Kohlenwasserstoffe. Besonders eigenen sich aromatische halogenierte Kohlenwasserstoffe und ganz besonders eignen sich Aromaten mit Alkygruppen.

Bei Durchführung in Lösung sind Konzentrationen von > 1 M Cycloocten bevorzugt. Besonders bevorzugt sind Konzentrationen von 1 bis 2 M und ganz besonders bevorzugt sind Konzentrationen von 2 bis 4 M an Cycloocten, bezogen auf das Lösungsmittel.

Bei dem beschriebenen Verfahren wird der Katalysator bezogen auf die Menge an Cycloocten in Mengen von 5 bis 0,0001 mol-% eingesetzt. Bevorzugt sind Mengen von 2 bis 0,001 mol-% und besonders bevorzugt sind Mengen von 1 bis 0,5 mol-% Katalysator bezogen auf die Stoffmenge an eingesetztem Cycloocten.

Um die α,β-ungesättigte Dicarbonsäure in Polymergrade-Qualität zu erhalten, bietet sich die Aufreinigung über Kristallisation, Destillation oder eine Kombination von beiden an.

Als Katalysatoren eignen sich Ruthenium-carben Komplexe die als eines der charakteristischen Merkmale einen N-heterocyclischen Carben Liganden tragen. Beispiele bevorzugter Katalysatoren finden sich in **Bild 1**. Besonders bevorzugt sind dabei Katalysatoren des Typs 7, mit einer Elektronen-ziehenden Gruppe R' am Benzylidenliganden.

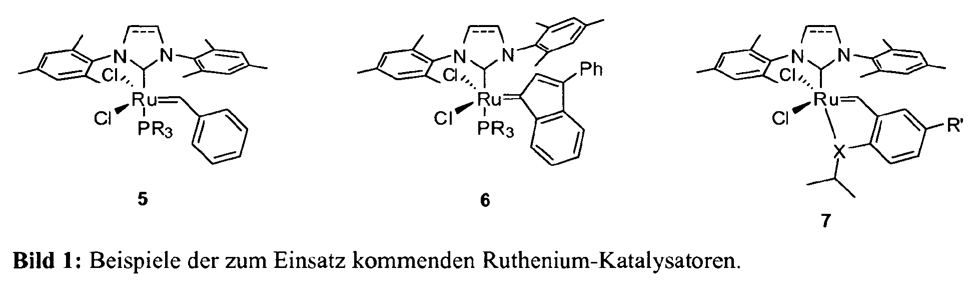

### Beispiele:

1. 1,3-Bis(2,4,6-trimethylphenyl)-4,5-dihydroimidazol-2-ylidene[2-(i-propoxy)-5-(N,N-dimethylaminosulfonyl)phenyl]methylenruthenium(II) dichloride (0,333 g, 0,45 mmol) wird in einem Dreihalskolben mit Rückflusskühler in Acrylsäure (13,09 g, 0,182 mol) unter Argon vorgelegt und auf 60 °C erhitzt. Cycloocten (10 g, 0,091 mol) wird über einen Zeitraum von 20 Minuten zugetropft. Nach 1,5 Stunden lässt man das Reaktionsgemisch auf Raumtemperatur abkühlen und versetzt mit Toluol (200 ml). Es wird kurz zum Sieden erhitzt und im Anschluss unter Rühren langsam abgekühlt. Der dabei ausfallende Feststoff wird abfiltriert und mit kaltem Toluol (3x 10 ml) gewaschen. Das Produkt wird nach Trocknung im Vakuum als weißer Feststoff erhalten (10,2 g, 50 %). Laut NMR-Analyse hat das Produkt eine Reinheit von > 99 %.
2. Ein Gemisch aus Cycloocten (10 g, 0,091 mol) und Acrylsäure (13,09 g, 0,182 mol) in Toluol (20 ml) wurde auf 60 °C erwärmt. Von einer Lösung von 1,3-Bis(2,4,6-trimethylphenyl)-4,5-dihydroimidazol-2-ylidene[2-(i-propoxy)-5-(N,N-dimethylamino-sulfonyl)phenyl]methylenruthenium(II) dichloride (0,667 g, 0,00091 mol) in Toluol (25 ml) werden zunächst 10 ml zugesetzt und die restlichen 15 ml über einen Zeitraum von einer Stunde zugetropft. Nach vollständiger Zugabe wird eine Stunde bei gegebener Temperatur gerührt und dann abgekühlt. Der ausgefallene Feststoff wird abfiltriert und mit kaltem Toluol (3x 5 ml) gewaschen. Das Produkt wird nach Trocknung im Vakuum als weißer Feststoff erhalten (10,2 g, 50 %). Laut NMR-Analyse hat das Produkt eine Reinheit von > 99 %.

## Patentansprüche

1. Verfahren zur Herstellung von Dodeca-2,10-dien-1,12-dicarbonsäure,
**dadurch gekennzeichnet,**
**dass** Cycloocten und Acrylsäure mit einem Ruthenium-Katalysator über eine Metathese-Reaktion bei hohen Substratkonzentrationen von 1 Mol/L Cycloocten bis hin zu Reaktionen in Substanz umgesetzt werden, wobei die dabei entstehende Dicarbonsäure ausfällt.

2. Verfahren nach Anspruch 1,
**dadurch gekennzeichnet,**
**dass** die erhaltene ungesättigte Dodeca-2,10-dien-1,12-dicarbonsäure in einem zweiten Schritt zur 1,12-Dodecandisäure hydriert wird.

3. Verfahren nach Anspruch 1,
**dadurch gekennzeichnet,**
**dass** bei der Reaktionsdurchführung in Lösung Cycloocten in Konzentrationen von 2 bis 4 Mol/L vorliegt.

4. Verfahren nach einem der vorhergehenden Ansprüche,
**dadurch gekennzeichnet,**
**dass** als Katalysatoren Ruthenium-carben Komplexe, die einen N-heterocyclischen Liganden tragen eingesetzt werden.

5. Verfahren nach einem der vorhergehenden Ansprüche,
**dadurch gekennzeichnet,**
**dass** die Ruthenium-Katalysatoren in Mengen von 5 bis 0,0001 mol-% bezogen auf die Stoffmenge an Cycloocten eingesetzt werden.

6. Verfahren nach einem der vorhergehenden Ansprüche,
**dadurch gekennzeichnet,**
**dass** die Ruthenium-Katalysatoren in Mengen von 2 bis 0,001 mol-% bezogen auf die Stoffmenge an Cycloocten eingesetzt werden.

7. Verfahren nach einem der vorhergehenden Ansprüche,
**dadurch gekennzeichnet,**
**dass** die Ruthenium-Katalysatoren in Mengen von 1 bis 0,5 mol-% bezogen auf die Stoffmenge an Cycloocten eingesetzt werden.

8. Verfahren nach einem der vorhergehenden Ansprüche,
**dadurch gekennzeichnet,**
**dass** als Lösungsmittel acyclische als auch cyclische Kohlenwasserstoffe eingesetzt werden.

9. Verfahren nach einem der vorhergehenden Ansprüche,
**dadurch gekennzeichnet,**
**dass** als Lösungsmittel aromatische halogenierte Kohlenwasserstoffe eingesetzt werden.

10. Verfahren nach einem der vorhergehenden Ansprüche,
**dadurch gekennzeichnet,**
**dass** die im Filtrat gelösten Ruthenium-Katalysatoren recycelt werden.

11. Verfahren nach einem der vorhergehenden Ansprüche,
**dadurch gekennzeichnet,**
**dass** die erhaltene ungesättigte Dicarbonsäure über Kristallisation, Destillation oder eine Kombination von beiden aufgereinigt wird.

## Claims

1. Process for preparing dodeca-2,10-diene-1,12-dicarboxylic acid,
**characterized in that**
cyclooctene and acrylic acid are reacted by means of a metathesis reaction in the presence of a ruthenium catalyst at high substrate concentrations of 1 mol/l of cyclooctene up to reactions in bulk, with the dicarboxylic acid formed precipitating.

2. Process according to Claim 1,
**characterized in that**
the unsaturated dodeca-2,10-diene-1,12-dicarboxylic acid obtained is hydrogenated to 1,12-dodecanedicarboxylic acid in a second step.

3. Process according to Claim 1,
**characterized in that**
cyclooctene is present in concentrations of from 2 to 4 mol/l when the reaction is carried out in solution.

4. Process according to any of the preceding claims,
**characterized in that**
ruthenium-carbene complexes which bear an N-heterocyclic ligand are used as catalysts.

5. Process according to any of the preceding claims,
**characterized in that**
the ruthenium catalysts are used in amounts of from 5 to 0.0001 mol% based on the molar amount of cyclooctene.

6. Process according to any of the preceding claims,
**characterized in that**
the ruthenium catalysts are used in amounts of from 2 to 0.001 mol% based on the molar amount of cyclooctene.

7. Process according to any of the preceding claims,
**characterized in that**
the ruthenium catalysts are used in amounts of from 1 to 0.5 mol% based on the molar amount of cyclooctene.

8. Process according to any of the preceding claims,
**characterized in that**
acyclic or cyclic hydrocarbons are used as solvents.

9. Process according to any of the preceding claims,
**characterized in that**
aromatic halogenated hydrocarbons are used as solvents.

10. Process according to any of the preceding claims,
**characterized in that**
the ruthenium catalysts dissolved in the filtrate are recycled.

11. Process according to any of the preceding claims,
**characterized in that**
the unsaturated dicarboxylic acid obtained is purified by crystallization, distillation or a combination of the two.

## Revendications

1. Procédé pour la préparation d'acide dodéca-2,10-diène-1,12-dicarboxylique, **caractérisé en ce qu'**on transforme du cyclooctène et de l'acide acrylique avec un catalyseur à base de ruthénium via une réaction de métathèse à des concentrations élevées en substrat de 1 mole/1 de cyclooctène jusqu'aux réactions en masse, l'acide dicarboxylique qui se forme précipitant.

2. Procédé selon la revendication 1, **caractérisé en ce que** l'acide dodéca-2,10-diène-1,12-dicarboxylique insaturé obtenu est hydrogéné dans une deuxième étape en acide 1,12-dodécanedioïque.

3. Procédé selon la revendication 1, **caractérisé en ce que**, lors de la réalisation de la réaction en solution, le cyclooctène se trouve en des concentrations de 2 à 4 moles/l.

4. Procédé selon l'une quelconque des revendications précédentes, **caractérisé en ce qu'**on utilise, comme catalyseurs, des complexes qui portent un ligand N-hétérocyclique.

5. Procédé selon l'une quelconque des revendications précédentes, **caractérisé en ce que** les catalyseurs à base de ruthénium sont utilisés en des quantités de 5 à 0,0001% en mole par rapport à la quantité molaire de cyclooctène.

6. Procédé selon l'une quelconque des revendications précédentes, **caractérisé en ce que** les catalyseurs à base de ruthénium sont utilisés en des quantités de 2 à 0,001% en mole par rapport à la quantité molaire de cyclooctène.

7. Procédé selon l'une quelconque des revendications précédentes, **caractérisé en ce que** les catalyseurs à base de ruthénium sont utilisés en des quantités de 1 à 0,5% en mole par rapport à la quantité molaire de cyclooctène.

8. Procédé selon l'une quelconque des revendications précédentes, **caractérisé en ce qu'**on utilise comme solvant des hydrocarbures acycliques ainsi que cycliques.

9. Procédé selon l'une quelconque des revendications précédentes, **caractérisé en ce qu'**on utilise comme solvants des hydrocarbures aromatiques halogénés.

10. Procédé selon l'une quelconque des revendications précédentes, **caractérisé en ce que** les catalyseurs à base de ruthénium dissous dans le filtrat sont recyclés.

11. Procédé selon l'une quelconque des revendications précédentes, **caractérisé en ce que** l'acide dicarboxylique insaturé obtenu est purifié par cristallisation, distillation ou une combinaison des deux.
